# EUROPEAN PATENT SPECIFICATION

(11) **EP 3 583 072 B1**
(45) Date of publication and mention of the grant of the patent: **23.11.2022**
(21) Application number: 18754439.0
(22) Date of filing: 15.02.2018
(51) Int. Cl.: C02F 1/32, A61L 2/10

(54) **METHOD FOR WATER DISINFECTION**
VERFAHREN ZUR DESINFEKTION VON WASSER
PROCÉDÉ DE DÉSINFECTION DE L'EAU

(30) Priority: 15.02.2017 US 201762459228 P
(43) Date of publication of application: 25.12.2019
(73) Proprietor: Ramot at Tel-Aviv University Ltd., 6139201 Tel-Aviv (IL)
(72) Inventor: GERCHMAN, Yoram, 3609628 Kiryat Tivon (IL); MAMANE, Hadas, 3088900 Caesarea (IL); COHEN YANIV, Vered, 6139201 Tel-Aviv (IL); BETZALEL, Yifaat, 6139201 Tel-Aviv (IL); BALAS, Avraham, 6139201 Tel-Aviv (IL)
(74) Representative: De Clercq & Partners
(86) International application number: PCT/IL2018/050174
(87) International publication number: WO 2018/150425

(56) References cited:
- EP-A1- 2 965 766
- EP-A1- 2 965 766
- WO-A1-2017/003347
- DE-A1-102010 005 893
- US-A1- 2005 242 013
- US-A1- 2009 250 626
- US-A1- 2013 236 353
- US-A1- 2014 166 897
- US-A1- 2015 314 024
- US-A1- 2016 083 272
- US-A1- 2016 271 280
- US-B2- 8 529 770

## Description

### TECHNICAL FIELD

The present invention provides a method for water disinfection. Further disclosed herein, but not according to the invention, is a water disinfection apparatus for carrying out said method.

### BACKGROUND ART

Ultraviolet (UV) irradiation is a well-established practice for water disinfection of pathogens. The UV spectrum is divided into UVC (200-280 nm), UVB (280-315 nm), and UVA (315-400 nm). Currently, two types of mercury vapor-filled lamps produce germicidal UV irradiation which are either nearly monochromatic at about 254nm (253.7nm) (low pressure/low pressure high output, LP/LPHO), or polychromatic (medium pressure, MP) that emit light at multiple peaks from 200 nm and above, including at 254nm. Full scale water disinfection UV-based systems vary in size, configuration (horizontal or vertical to the flow), lamp type (LP or MP), UV transmittance (UVT) sensors, and reactor design (in-line reactors or submerged lamps in open conduit) (Water Environment Federation, 2015).

Germicidal UV action refers to the wavelengths of UV irradiation that induce photochemical reactions effective in microbial inactivation. Nucleic acids show significant absorption of UV photons between 200 and 300 nm, with peak absorption at 265 nm. A UV action spectrum is determined by measuring the dose response of a microorganism to various wavelengths. This action spectrum highly depends on the wavelength and may vary between different microorganisms. The absorbance spectrum of an isolated DNA of a particular microorganism may differ from the actual action spectrum, because photons can be absorbed in other cell components as shown for *Bacillus subtilis* spores (Chen *et al.,* 2009; Mamane-Gravetz *et al.,* 2005). Thus, calculating a MP UV average germicidal dose based on DNA absorption (typically determined for *Escherichia coli;* Meulemans, 1987), with relative peak sensitivities in the 260-265nm region, does not account for non-DNA-based damages.

The power input of LP lamps or LPHO measured per lamp (100W and 150-500W, respectively) is lower than for MP lamps (3000-5000W), and the number of LP lamps required so as to provide a similar wattage is thus higher. Nevertheless, the germicidal efficiency, i.e., the conversion efficiency of electrical power to UVC photons in the absorbance spectrum of DNA for LP lamps, is higher than that for MP lamps, and therefore more electrical energy is required by MP lamps to emit the same UV energy as LP lamps.

MP lamps are disadvantageous over LP/LPHO lamps because they are more expensive; have lower germicidal efficiency (12-16%) compared to LPHO (30-35%); have higher operating temperature (600-950°C) compared to LPHO (150-200°C), which results in higher fouling rate on the lamp sleeves (removal of the fouling requires cleaning by mechanical (wipers) and chemical (acid) methods and disposal of chemical waste, thus increasing the maintenance cost); have a shorter life time (8000 hours) compared to LP/LPHO (12,000-16,000 hours); and emit wavelengths that are non-usable for disinfection, e.g., in the infrared (IR) and longer warm up time. However, MP lamps have still advantageous considering that they target cellular components other than the DNA, such as proteins (various proteins and enzymes were found to absorb UVB and UVC, resulting in further bacterial damage (Harm, 1980; Oguma *et al.,* 2002; Sinha and Häder, 2002). Proteins typically show an absorption peak around 280nm, with a minimum around 240-250 nm (Jagger, 1967); and that less lamps are required compared to LP so as to achieve the same effect.

As stated above, the germicidal action spectrum of a UV light may depend on the microorganism. For example, while organisms such as *E. coli, Salmonella typhimurium, Pseudomonas aeruginosa, B. subtilis, Cryptosporidium parvum* oocysts, and *Bacillus pumilus* have relative peak sensitivities in the range of 260-265 nm (Beck *et al.,* 2015; Chen *et al.,* 2009; Gates, 1930; Lakretz *et al.,* 2010; Linden *et al.,* 2001; Mamane-Gravetz *et al.,* 2005; Wang *et al.,* 2005), *Herpes simplex* virus exhibits peak sensitivities in the range of 270-280 nm (Detsch *et al.,* 1980). The maximum sensitivity of various microorganisms thus extends beyond the 254nm emitted by LP lamps. An additional issue is the bacterial recovery following exposure to mercury UV lamps. Zimmer and Slawson (2002) showed that *E. coli* underwent photorepair following exposure to LP, but no repair was detected following exposure to the MP lamp (10 mJ/cm²).

MP systems are typically equipped with automatic wipers to address fouling of quartz sleeves. Occasionally, the automatic cleaning is not sufficient, and this results in increase in the intensity of the lamps (the UV sensor senses a decrease in lamp intensity and thus increases the intensity of the lamp to compensate), and consequently in increase in the system electrical consumption and decrease in the disinfection effectiveness. The above results in temperature increase followed by inorganic precipitation, up to a situation where the transmitted irradiation is so low that the system must be disabled to perform chemical acid cleaning. Precipitation of the MP lamp sleeve is more severe compared to LPHO lamp, as MP lamp temperature reaches 950°C compared to 150-200°C in LPHO, and chemical acid cleaning for MP lamps is therefore required more frequently than for LPHO lamps. Accordingly, the US Environmental Protection Agency (USEPA) (2006) guidelines for UV specifically recites: "Compounds for which the solubility decreases as temperature increases may precipitate (e.g., CaCO₃, CaSO₄, MgCO₃, MgSO₄, FePO₄, FeCO₃, Al₂(SO₄)₃) and foul MP lamps faster than LP or LPHO lamps because MP lamps operate at higher temperatures".

UV light-emitting diodes (LEDs) are considered as alternatives to UV mercury lamps in water treatment. These UV sources allow flexible design (point source vs. cylindrical tube in LP and MP) and construction of UV reactors, and enables tuning the wavelength; require no lamp warm-up time; can be operated by intermittent flow and at ambient temperature, thus do not promote fouling; have lower electricity consumption; and require a DC voltage thus can be powered with a battery/solar cell. In contrast to mercury lamps, LEDs are safe to dispose (Chen *et al.,* 2017).

UVC-LEDs have shown ability to inactivate bacteria as *E. coli,* spores of *B. subtilis* and bacteriophages (MS2) (Chen *et al.,* 2017). However, this technology is currently limited due to low power input and radiant flux, which limit the efficiency to a few percentages only, short life-time, and extremely high costs, and it is therefore immature for industrial use in water disinfection. US 2009/250626, US 2014/166897 and US 2016/271280 disclose methods for water disinfection comprising exposing the water simultaneously to UV radiation of two different wavelengths.

### SUMMARY OF INVENTION

The present invention relates to a method for water disinfection according to claim 1. In particular embodiments, at least one UV irradiation source comprises a LP UV irradiation source emitting light at a wavelength of about 254nm, or a UV LED irradiation source emitting light at a wavelength of between 260nm and 270nm.

In another aspect, not according to the invention is herein disclosed a water disinfecting apparatus comprising: (i) a chamber having an inlet adapted for connection to a pressurized water source, and an outlet; (ii) at least one UV irradiation source emitting light at a wavelength of between 250nm and 280nm; and (iii) at least one UV irradiation source emitting light at a wavelength of between 285nm and 310nm, wherein said UV irradiation sources are designed/configured to emit UV light into said chamber when water passes therethrough. Particular such apparatus are those wherein said at least one UV irradiation source emitting light at a wavelength of between 250nm and 280nm comprises a LP UV irradiation source emitting light at a wavelength of about 254nm, or a UV LED irradiation source emitting light at a wavelength of between 260nm and 270nm; and said at least one UV irradiation source emitting light at a wavelength of between 285nm and 310nm comprises a UV LED irradiation source emitting light at a wavelength of between 290nm and 300nm.

### BRIEF DESCRIPTION OF DRAWINGS

**Fig. 1** shows spectral emission of MP and LP UV lamps.
**Figs. 2A-2B** show a schematic diagram of the LP-LED collimated beam apparatus described in the Experimental setup below, including a mercury LP lamp and a LED array, and an electromagnetic peripheral source for stirring the water sample **(2A);** and the stirring device designed to allow effective mixing of the water sample irradiated **(2B).**
**Fig. 3** shows *SodA* gene activation in *E. coli* by exposure to MP lamp (right); and the plasmid with lux genes fused to the *SodA* promoter used (left).
**Fig. 4** shows *E. coli* germicidal inactivation by LP and MP lamps, where the average irradiation was compared and weighted between wavelengths of 220-300nm and 250-262nm (the range in which the two lamps emit).
**Fig. 5** shows log inactivation of *E. coli* MG1655 strain vs. irradiation time, by a UV LED irradiation source emitting light in the 285-305 nm range, a LP irradiation source emitting light of about 254nm, and the combination thereof, compared to the theoretical value representing the LP+LED summation.
**Fig. 6** shows log inactivation of *E. coli* MG1655 strain vs. germicidal dose, by a combination of LP+ UV LED irradiation source emitting light in the 285-305 nm range, compared to the theoretical value representing the LP+LED summation.
**Fig. 7** shows log inactivation of natural indigenous coliforms in wastewater secondary effluent by LP or LP+UV LED in the 285-305 nm range.
**Fig. 8** illustrates a water disinfecting apparatus having a tube/cylindered-shaped chamber.
**Fig. 9** illustrates a cross-section view of the water disinfecting apparatus illustrated in Fig. 8, showing the location of the inner LP UV lamp along the centerline radius of the tube, while the LED UV lamps are positioned around and across the perimeter of said tube.
**Fig. 10** illustrates a cross-section view of the water disinfecting apparatus illustrated in Fig. 8, and further illustrates that the surroundings of the transparent regions at the tube are designed to enable easy mounting and replacing of an individual LED array.
**Fig. 11** illustrates various ultraviolet reactor baffle designs for advanced ultraviolet disinfection showing various ways to increase turbulence of the water passing within the tube.

### DETAILED DESCRIPTION

It has now been found in accordance with the present invention that while photons from LP UV lamp target mainly DNA, resulting in formation of nucleotide dimers, irradiation of bacteria with photons at wavelengths in the UVB spectrum, more specifically between 285nm and 305nm, results in the production of superoxide radicals inside the bacterial cell, most likely due to photoactivation of aromatic amino acids and mainly tryptophan. Such radicals can target cellular components other than DNA, e.g., cell membrane, small molecules, and proteins that are essential for almost all cell activities, including the repair of DNA dimers.

As shown herein, the combination of DNA damaging photons, i.e., photons emitted from an irradiation source emitting light at a wavelength in the range of 250-280nm, e.g., a LP UV lamp emitting light at a wavelength of about 254nm or a UV LED emitting light at a wavelength in the range of 260-270nm; with superoxide generating photons, i.e., photons emitted from an irradiation source emitting light at a wavelength of 285-310nm, e.g., UVB LED lamp, provides a synergistic effect and results in much more effective inactivation of pathogens and lower recovery thereof. For example, a combination of a LP UV lamp emitting light at a wavelength of about 254nm and a UV LED emitting light at a wavelength of 285-305nm resulted in enhanced and synergistic inactivation of *E. coli* strains compared to either one of said irradiation sources and compared to the theoretical value representing the LP+LED summation, wherein germicidal efficiency is increased exponentially with increase in the irradiation time.

While MP UV lamps require much more electricity and produce much more heat, resulting in precipitation of scaling on the quartz sleeve covering the lamp and the need for periodic complex and expensive cleaning, a combined system as shown herein will have both the benefits of LP/LPOH, i.e., low power consumption and low heat production, and of MP systems, i.e., better inactivation, and less recovery; and could be used for inactivation of microorganisms in a variety of applications, including water and air disinfection.

A combined apparatus as disclosed herein will thus achieve MP level inactivation of waterborne pathogens while avoiding the disadvantages of current MP lamps **(Table 1),** wherein careful selection of the LEDs wavelength(s) could be chosen to damage the pathogen by additional mechanisms (production of superoxide radicals damaging cellular components other than the DNA, etc.) resulting in a synergistic effect. Furthermore, the modular design of the apparatus could offer easy tailoring of the apparatus (and the method using said apparatus) for specific pathogens by choosing the required LEDs and replacing them as needed.

The present invention thus relates to a method for water disinfection comprising exposing water, simultaneously, to a combination of at least two UV irradiation sources, for a sufficient period of time, wherein at least one of said UV irradiation sources emits light at a wavelength of between 260nm and 275nm, and at least one of said UV irradiation sources emits light at a wavelength of between 295nm and 305nm.

The phrase "UV irradiation source emitting light at a wavelength of between 250nm and 280nm" means a monochromatic (or nearly monochromatic) UVC irradiation source having a definite wavelength between 250nm and 280nm, or a polychromatic UVC irradiation source with emission wavelength, but preferably with peak emission wavelength, between 250nm and 280nm.

The phrase "UV irradiation source emitting light at a wavelength of between 285nm and 310nm" means a monochromatic (or nearly monochromatic) UVB irradiation source having a definite wavelength between 285nm and 310nm, or a polychromatic UVB irradiation source with emission wavelength, but preferably with peak emission wavelength, between 285nm and 310nm.

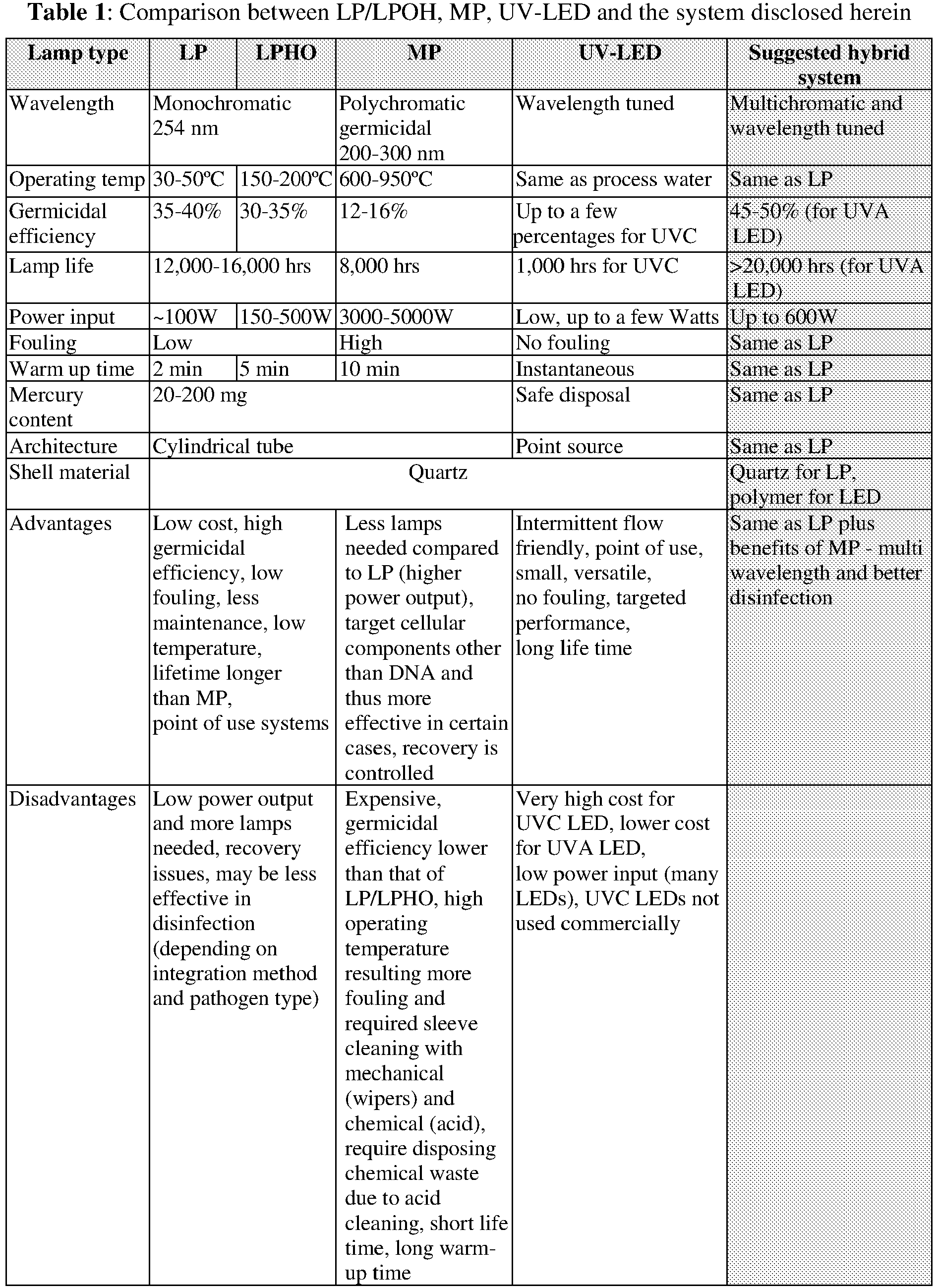

In certain embodiments, said at least one UV irradiation source emitting light at a wavelength of between 260nm and 275nm and utilized according to the method of the present invention comprises a sole UV irradiation source, or a plurality of UV irradiation sources emitting light at either the same or different wavelengths between 260nm and 275nm. In certain particular such embodiments, said at least one UV irradiation source comprises a low pressure (LP) UV irradiation source, e.g., a LP UV irradiation source emitting light at a wavelength of about 254nm. In other particular such embodiments, said at least one UV irradiation source comprises at least one UV light-emitting diode (LED) irradiation source each independently emitting light at a wavelength of between 260nm and 275nm, or between 260nm and 270nm, e.g., at a wavelength of about 260nm, about 261nm, about 262nm, about 263nm, about 264nm, about 265nm, about 266nm, about 267nm, about 268nm, about 269nm, or about 270nm.

In certain embodiments, said at least one UV irradiation source emitting light at a wavelength of between 295nm and 305nm and utilized according to the method of the present invention comprises a sole UV LED irradiation source or a plurality of UV LED irradiation sources/array emitting light at either the same or different wavelengths between 295nm and 305nm. In particular such embodiments, each one of said UV LED irradiation sources independently emits light at a wavelength of between 295nm and 300nm or between 300nm and 305nm, e.g., at a wavelength of about 295nm, about 296nm, about 297nm, about 298nm, about 299nm, or about 300nm.

According to the method of the present invention, the water treated, i.e., undergoing disinfection, are exposed, for a sufficient period of time, to a combination of at least two UV irradiation sources as defined in any one of the embodiments above. According to the invention, the exposure of said water to said at least two UV irradiation sources is carried out simultaneously.

The phrase "sufficient period of time" as used herein means a period of time that is sufficient to disinfect the water treated according to the method of the invention. Such a period of time may vary depending on various parameters such as the type/nature of the water treated (drinking water or wastewater effluents of different origins); water quality parameters including UV transmittance (UVT); water flow rate; the microbial pathogens characterizing, i.e., found in, said water; geographic-related conditions; and designated use. A period of time sufficient for disinfecting the water treated by this method may range from a few seconds, e.g., 1, 2, 3, 4, 5, 6, 7, 8, 9 or 10 seconds, and up to a few minutes, e.g., 1, 2, 3, 4, 5 or 6 minutes, but it preferably ranges from a few seconds and up to several tens of seconds, i.e., up to 10, 20, 30, 40, 50, 60, 70, 80, 90, 100, 110 or 120 seconds. Yet, it may be assumed that the period of time required for disinfecting said water, when exposed to said at least two UV irradiation sources sequentially, would be longer than that required when said water are exposed to said UV irradiation sources simultaneously.

In certain embodiments, the present invention relates to a method for water disinfection as defined in any one of the embodiments above, wherein at least one UV irradiation source comprises a LP UV irradiation source emitting light at a wavelength of about 254nm; or a UV LED irradiation source emitting light at a wavelength of between 260nm and 275nm, or between 260nm and 270nm, e.g., at a wavelength of about 260nm, about 261nm, about 262nm, about 263nm, about 264nm, about 265nm, about 266nm, about 267nm, about 268nm, about 269nm, or about 270nm; and at least one UV irradiation source comprises a UV LED irradiation source emitting light at a wavelength of between 295nm and 300nm or between 300nm and 305nm, e.g., at a wavelength of about 295nm, about 296nm, about 297nm, about 298nm, about 299nm, or about 300nm. According to the invention, the exposure of said water to said at least two UV irradiation sources is carried out simultaneously.

In another aspect, not according to the invention is disclosed herein an apparatus **200,** or system, for carrying out the method of the invention, more particularly, a water disinfecting apparatus **200** that enables simultaneous irradiation from two directions while mixing the passing water. This apparatus **200** comprises (i) a chamber **201,** e.g., a vessel or reactor, having an inlet **202** adapted for connection **201** to a pressurized water source, and an outlet **203;** (ii) at least one UV irradiation source **207** emitting light at a wavelength of between 250nm and 280nm; and (iii) at least one UV irradiation source emitting light at a wavelength of between 285nm and 310nm, wherein said UV irradiation sources are designed/configured to emit UV light into said chamber **201** when water passes therethrough.

Disclosed herein is a system as defined above, wherein said at least one UV irradiation source **207** emitting light at a wavelength of between 250nm and 280nm comprises a sole UV irradiation source **207** or a plurality of UV irradiation sources emitting light at either the same or different wavelengths between 250nm and 280nm. Particular such systems are those wherein said at least one UV irradiation source comprises a LP UV irradiation source **207,** e.g., a LP UV irradiation source emitting light at a wavelength of about 254nm. Also disclosed herein is a system as defined above, wherein said at least one UV irradiation source comprises at least one UV LED irradiation source each independently emitting light at a wavelength of between 260nm and 275nm, between 260nm and 270nm, or between 260nm and 265nm.

Disclosed herein is a system as defined above, wherein said at least one UV irradiation source emitting light at a wavelength of between 285nm and 310nm comprises a sole UV LED irradiation source or a plurality/array of UV LED irradiation sources **205** emitting light at either the same or different wavelengths between 285nm and 310nm. In the system as disclosed above, each one of said UV LED irradiation sources independently emits light at a wavelength of between 285nm and 290nm, between 290nm and 295nm, between 295nm and 300nm, between 300nm and 305nm, or between 305nm and 310nm.

Disclosed herein is a system as defined above, wherein (i) said at least one UV irradiation source **207** emitting light at a wavelength of between 250nm and 280nm comprises LP UV irradiation source emitting light at a wavelength of about 254nm; or a UV LED irradiation source emitting light at a wavelength of between 260nm and 275nm, or between 260nm and 270nm, e.g., at a wavelength of about 260nm, about 261nm, about 262nm, about 263nm, about 264nm, about 265nm, about 266nm, about 267nm, about 268nm, about 269nm, or about 270nm; and (ii) said at least one UV irradiation source emitting light at a wavelength of between 285nm and 310nm comprises a UV LED irradiation source **205** emitting light at a wavelength of between 285nm and 290nm, between 290nm and 295nm, between 295nm and 300nm, between 300nm and 305nm, or between 305nm and 310nm, e.g., at a wavelength of about 290nm, about 291nm, about 292nm, about 293nm, about 294nm, about 295nm, about 296nm, about 297nm, about 298nm, about 299nm, or about 300nm.

The water disinfecting apparatus **200** as disclosed above comprises a chamber **201** having an inlet **202** adapted for connection to a pressurized water source, and an outlet **203;** as well as at least two UV irradiation sources each as defined in any one of the embodiments above, configured to emit UV light into said chamber **201** when water passes therethrough. Said chamber **201** may be configured, e.g., as a tube.

The system disclosed herein comprises a chamber 201 configured as a tube, wherein (i) said at least one UV irradiation source **207** emitting light at a wavelength of between 250nm and 280nm is located across the center of said tube; and (ii) said at least one UV irradiation source emitting light at a wavelength of between 285nm and 310nm is a single UV LED or UV LED array(s) **205** located at the perimeter of said tube, e.g., distributed around and across the perimeter of said tube.

Said tube is either made of, or comprises at least one region **204** made of, a material transparent to said UV LED irradiation of a wavelength of between 285nm and 310nm; and said at least one UV LED irradiation source emitting light at a wavelength of between 285nm and 310nm is located externally to said tube or said at least one region **204,** respectively, such that it does not come in contact with the water when passes through said tube. In alternatives, said material transparent to said UV LED irradiation of a wavelength of between 285nm and 310nm is not transparent to UV irradiation of a wavelength of between 250nm and 280nm.

In other alternatives, said UV LED irradiation source emitting light at a wavelength of between 285nm and 310nm is located inside said tube and is waterproof. In yet other alternatives, said tube is made of opaque material or from material not transparent to the UV wavelengths emitted by either UV irradiation sources.

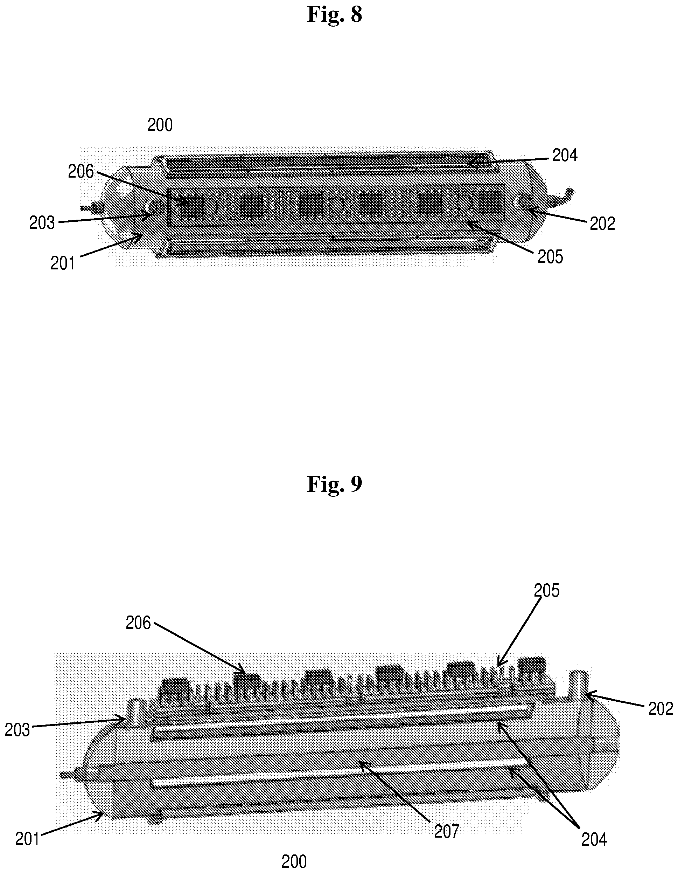

**Fig. 8** shows an illustration of a water disinfecting apparatus **200** having a tube/cylindered-shaped chamber **201.** **Fig. 9** illustrates a cross-section view of the water disinfecting apparatus **200** illustrated in **Fig. 8****,** showing the location of the inner LP UV lamp along the centerline radius of the tube, while the LED UV lamps/arrays **205** are positioned around and across the perimeter of said tube. Notably, **Figs. 8-9** illustrate a single LED array **205,** but it should be noted that similar arrays are positioned/evenly-distributed all-around said tube. As illustrated in **Fig. 8****,** the water disinfecting apparatus **200** may further comprise a cooling unit **206** for cooling the UV LED array **205** if needed or the water passing therein. Alternatively, the water disinfecting apparatus **200** may further comprise a heating unit, either instead or in addition to said cooling unit **206,** for heating the water passing therein, e.g. for increasing the disinfection process.

Notably, when the entire tube is made of a transparent material, said LED arrays **205** can be positioned at any location at the perimeter of the tube, wherein the areas in between said LED arrays **205** might be covered in order to prevent UV light from exiting the tube. Such covering may be done with either a reflective material/surface (as detailed herein below), or a light-absorbent material. Alternatively, when the tube comprises transparent regions **204** (as illustrated in **Figs. 8-10****),** said LED arrays **205** are positioned over said transparent regions **204** to enable UV light emitted therefrom to reach the water when it passes through said tube.

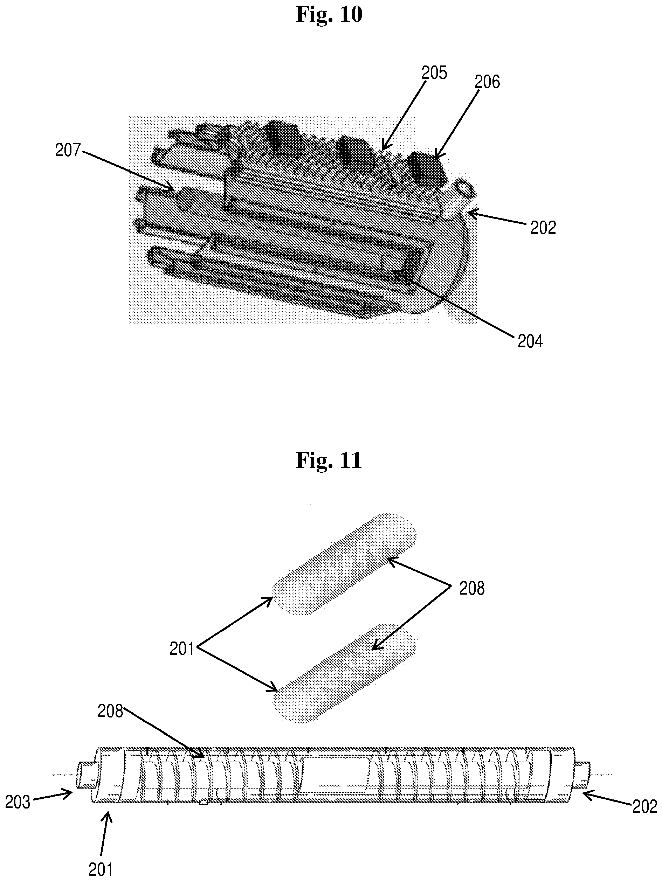

**Fig. 10** illustrates another cross-section view of the water disinfecting apparatus **200** illustrated in **Figs. 8-9****,** further illustrating that the surroundings of the transparent regions **204** at the tube are designed to enable easy mounting and replacing of an individual LED array **205.** As illustrated in **Fig. 10****,** each LED array **205** and transparent region **204** may comprise rails and grooves for sliding said LED array **205** on said transparent region **204,** and off when needed, e.g., for repair or changing the UV wavelength emitted by the LED array **205.**

In further alternatives, said at least one UV irradiation source **207** emitting light at a wavelength of between 250nm and 280nm is located in a UVC transparent sleeve located at the center along the axis of said tube, i.e., along the centerline radius. In more particular alternatives, the transparent sleeve is made of quartz, soda lime glass (also called soda-lime-silica glass), or a UVC-transparent polymer such as a polyacrylate (commonly known as acrylics). Non-limiting examples of polyacrylates include polyacrylamide, polyacrylonitrile, polymethylacrylate, polymethylmethacrylate (known as plexiglass), polyethylacrylate, polypropylacrylate, poly(2-ethylhexyl)acrylate, polyhydroxyethylmethacrylate, polybutylacrylate, and sodium polyacrylaye.

In still further particular alternatives, (i) said at least one UV LED irradiation source emitting light at a wavelength of between 285nm and 310nm is distributed around and across the perimeter of said tube, and is located externally such that it does not come in contact with the water when passes through said tube; and (ii) said at least one UV irradiation source **207** emitting light at a wavelength of between 250nm and 280nm is located in a transparent sleeve located at the center along the axis of said tube, wherein said transparent sleeve is made of quartz or a UVC-transparent polymer.

In alternatives, the water disinfecting apparatus **200** comprises a chamber **201** configured as a tube, according to any one of the alternatives above, wherein said tube further comprises reflective surface(s) positioned onto the inner surface of said tube, for increasing the effect of UV irradiation on the water when passes through said tube, due to total reflection (and not absorbance) of the UV photons at the walls. In such alternatives, said reflective surface(s) are made of polytetrafluoroethylene (Teflon), aluminum, stainless steel, or a refractive polymer, optionally coated for better reflectance. In other alternatives, said reflective surface(s) are made of Heraeus quartz materials such as HSQ100. In other alternatives, said reflective surfaces are positioned onto the outer surface of said tube, e.g., between the LED arrays **205.**

In alternatives, the water disinfecting apparatus **200** comprises a chamber **201** configured as a tube, according to any one of the alternatives above, wherein said tube further comprises baffles **208** to enhance radial mixing (whirling) of water when passes through said tube, and consequently increase the period during which said water is being exposed to said UV irradiation. Examples of a tube having such baffles **208** are illustrated in **Fig. 11****.**

Disclosed herein is a water disinfecting system according to any one of the alternatives above, wherein said system further comprises a controller configured to receive input indicating at least one of: (a) one or more flow characteristics of the water (e.g., flow rate); (b) water temperature (e.g., using thermocouple); (c) intensity of light emission from said UV irradiation sources; (d) water turbidity; and (e) water UV transmittance, wherein based on said input, said controller controls/adjusts the energy input of said UV irradiation sources and/or the water flow rate to thereby optimize water disinfection.

The technology being the basis for the method and the apparatus **200** disclosed herein provides a low cost and highly-efficient solution for water disinfection. This technology is based on a combined, simultaneous, use of a LP lamp, emitting a wavelength of about 254nm that is absorbed by DNA molecules, and UV LEDs, emitting light at a wavelength of between 295nm and 305nm, and providing a synergistic effect to the irradiation effect of the LP lamp.

It should be understood, that although UVC-LEDs currently exist, such LEDs are expensive, and both their external quantum efficiency and wall-plug efficiency are very low (below 5% and 1-3%, respectively) in comparison to LP UV lamps (about 35% wall-plug efficiency; Beck *et al.*, 2017), due to the low electrical conductivity of high aluminum composition AlGaN (aluminum gallium nitride). These limits make UVC-LEDs less practical as a replacement to LPUV lamps for the foreseen future (Moe, 2014). Indeed, the only UVC-LED based commercial water treatment device currently available is limited to a flow rate of 12 LPM, which is not realistic for any commercial scale water treatment that can be done using a combination of standard LDHF with matching LED lamps.

The term "about", when used in this specification with respect to a wavelength, means that the wavelength value recited may vary by up to plus or minus 0.5nm, e.g., a wavelength of about 254nm may practically vary from 253.5nm to 254.5nm.

The invention will now be illustrated by the following non-limiting Examples.

### EXAMPLES

### Experimental setup

### LP and MP exposures

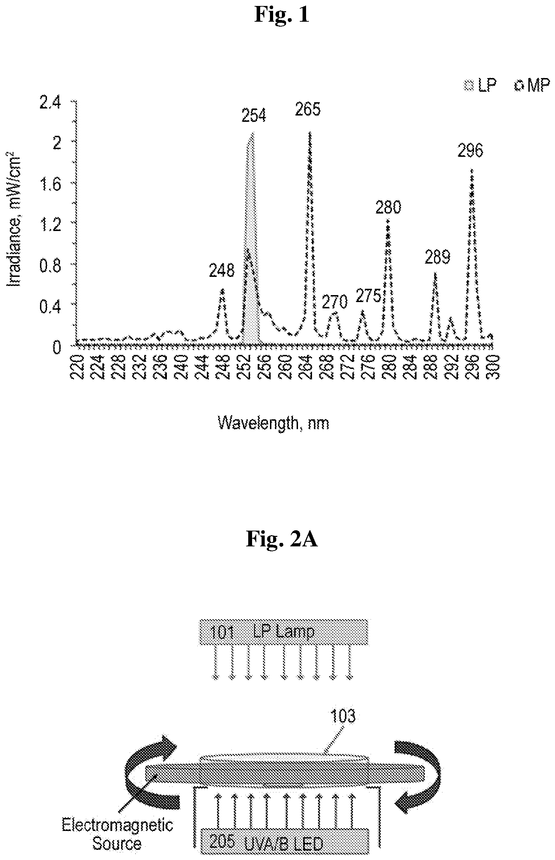

UV exposures were carried out using a medium pressure (MP) bench scale UV collimated beam apparatus. The UV irradiation was directed through a circular opening (collimated tube) to provide incident irradiation normal to the surface of the water test suspension. Emission spectrum of the LP and MP UV lamps are shown in **Fig. 1****.** LP lamps emit UV irradiation with a maximum peak at ~254nm (253.7nm), while MP lamps emit light at multiple peaks from 200 nm and above.

### Identifying ranges of wavelengths facilitating microorganism's inactivation

Here we examine which specific wavelengths of UVA/B-LED range are the most effective for integration with LP or LPHO mercury lamp (or LED emitting light at a wavelength of about 254nm) for inactivation and recovery control of various microorganisms.

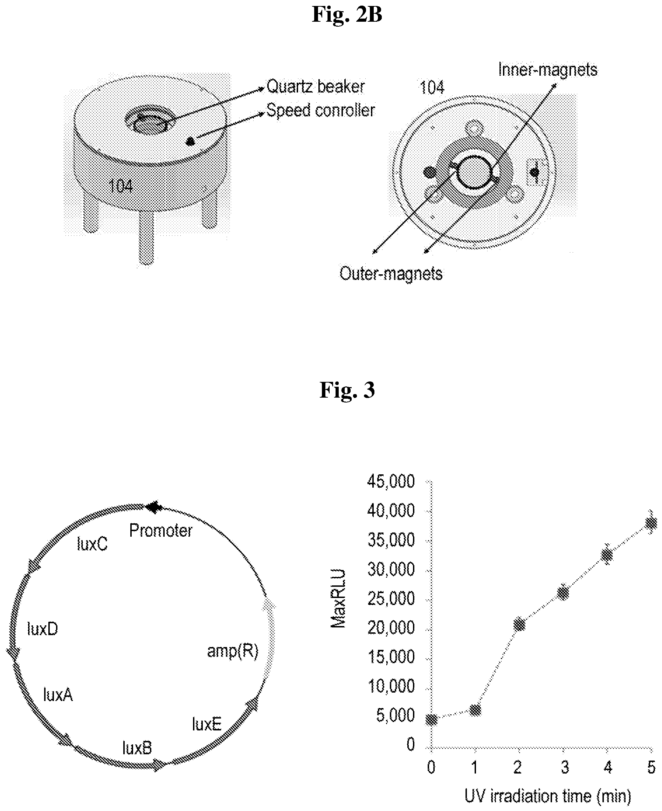

For this purpose, a unique LP-LED collimated beam apparatus was designed, that includes two UV sources emitting simultaneously: a mercury LP lamp **101** and a LED array **205,** where the LP lamp irradiation is directed through a collimator and irradiates incident to the vessel **103** with a liquid containing microorganisms (a petri dish), and a UVA/B-LED system simultaneously irradiates from the other side of the petri dish **103 (****Fig. 2A****).** To ensure mixing and uniformity, the liquid containing the microorganisms was continuously mixed using a unique stirring device **104** schematically shown in **Fig. 2B****,** which allows mixing using a magnet that moves at the periphery thus not obscuring the irradiation from either side.

### Inactivation experiments

To examine spiked *E. coli* and MS2 coliphage inactivation, aliquots of bacteria or bacteriophage were suspended in buffer at initial concentrations of about 10⁶ colony forming units (CFUs)/mL or plaque forming units (PFUs)/mL respectively. The microorganisms were exposed to a range of UV fluence by placing the suspension in a quartz dish and irradiating the samples while stirring. Irradiation was carried out using one or more of the following UV-sources: LP lamp and UV-LEDs. Each sample was serially diluted and enumerated to determine the bacterial (as CFUs) or bacteriophage number (as PFUs). Enumeration of spiked bacterial colonies was accomplished using the drop plate method (on LB plates) and enumeration of spiked bacteriophage plaques was accomplished using the double agar layer method (on tryptic soy agar (TSA) plates). Dose-response curves were developed by irradiating for different times, with microorganism concentration before UV exposure taken as the initial concentration, N₀, and arithmetic mean concentration per fluence as N_{D}. The log₁₀ transformation for N₀/N_{D} was plotted as a function of the average UV fluence.

The inactivation of secondary wastewater effluent containing indigenous bacteria was examined as well. To this end the water was placed in a quartz dish as above and irradiated with an LP, LED, or both, for the designated times. Water samples were taken, diluted (where needed) in sodium chloride saline solution and filtered on 0.2 µm filter. The filters were placed on m-ENDO agar LED plates and allowed to grow for 24h at 35°C. For coliforms bacteria enumeration, red colonies with green metal sheen were counted.

For *SodA* promoter activation experiments, *E. coli* MG1655 carrying a plasmid with lux genes fused to the *SodA* promotor was used **(****Fig. 3****).** The bacteria were grown in Luria Bertani (LB) broth supplemented with the proper antibiotic, washed twice, and resuspended in phosphate buffered solution (PBS); and were then irradiated as above, and transferred to 96-well plate. Concentrated LB was added, and the bio-luminescence was quantified over 10 hours in plate reader. Increase in luminesce values indicated the *sodA* promoter activation.

### Results and discussion

The efficiency of a hybrid system combining LP lamp with UVA/UVB wavelength from LEDs, as a replacement for MP lamps, was tested, wherein the LP lamp is used as a source for DNA-damaging 254nm irradiation, and the UVA/B LED is used as a source for supplementary wavelength disinfecting the pathogens via different mechanisms such as production of radicals.

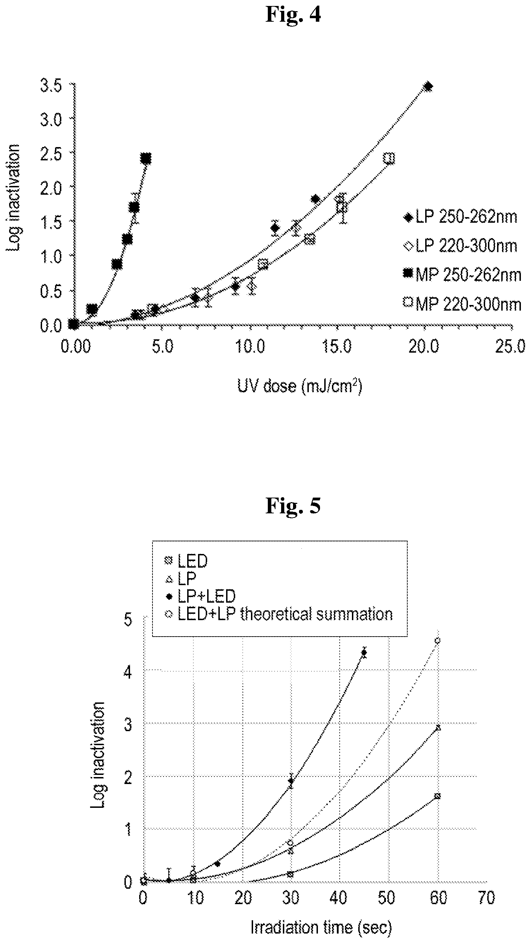

**Fig. 4** shows the *E. coli* germicidal inactivation vs. irradiation dose for LP and MP lamp. When the irradiation was integrated between wavelengths of 250-262nm (the range in which both lamps emit) the MP outperformed the LP, but when the 220-300nm was integrated no benefit was noted for the MP over the LP. As the MP and LP have similar integrated irradiation at a wavelength range of 250-262nm, the improved inactivation by the MP lamp demonstrates the importance of irradiation in wavelengths higher than the 262nm range.

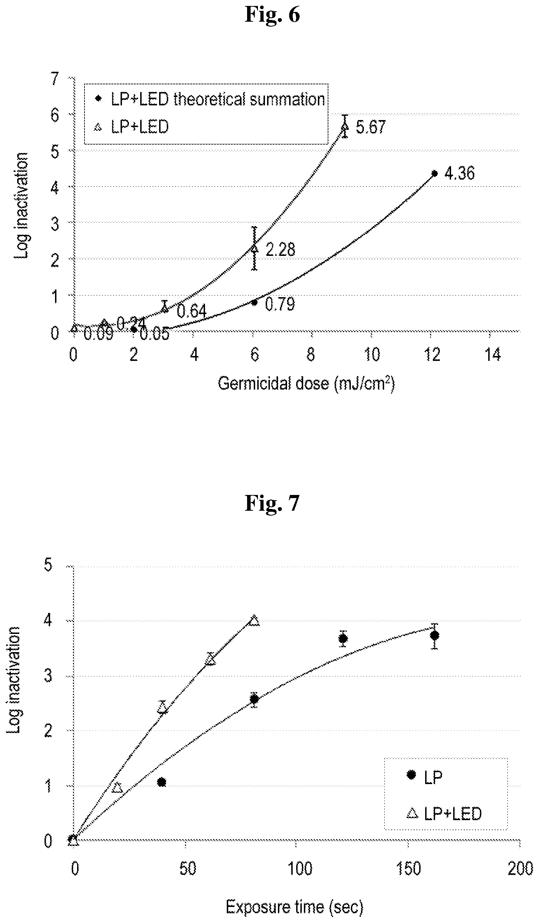

**Fig. 5** and **Fig. 6** show the inactivation curve of *E. coli* strain MG1655, by LP lamp or a UV LED emitting light in the 285-305 nm range, and the combination thereof, for various irradiation times and germicidal doses, respectively. The results demonstrate that the LP+LED combination results in enhanced and synergistic *E. coli* strain MG inactivation compared to either one of the two irradiation sources. Moreover, the germicidal efficiency of the combination was increased exponentially with increase in the irradiation time, in good agreement with involvement of more than one inactivation mechanism. The results shown illustrate that the combined LP+LED irradiation yields a maximum of 2.6 log over the theoretical additive effect of the two, and 3.5 log over LP only. Similar results were demonstrated for *E. coli* strain RFM443 (data not shown). The difference in inactivation vs. germicidal dose clearly demonstrates that mechanisms other than direct DNA damage are involved.

**Fig. 7** shows log inactivation of natural indigenous coliforms in wastewater secondary effluent by LP or LP+LED emitting light in the 285-305 nm range. The data shown illustrate that the LP+LED combination results in enhanced inactivation compared to the LP irradiation alone also for indigenous microorganisms. Inactivation of *Salmonella* and/or *Shigella* in the same wastewater effluent provided similar results (data not shown).

In order to understand what happens inside the bacteria under UV irradiation, bacteria with reporter genes (lux) fused to different promoters were used. Among others, we used the *sodA* promoter, that promotes the expression of the superoxide dismutase enzyme that catalyzes destruction of O₂⁻ radicals and protects cells against harmful effects of superoxide radicals (Lee and Gu, 2003). As shown in **Fig. 3****,** *sodA* gene was activated in *E. coli* bacteria exposed to MP lamp, indicating that exposure to MP lamp results in the formation of superoxide radical (O₂⁻ radicals) inside the bacteria, which could explain the more significant effect of MP lamps. Similarly to MP, irradiation with UV LED emitting light in the 285-305 nm range results in increase in the *sodA* promoter activation, suggesting that said irradiation generates superoxide radicals inside the cells (data not shown).

The results shown herein demonstrate that while photons from LP UV lamp target mainly DNA, irradiation of bacteria by photons at wavelengths longer than 254nm, such as emitted from a MP UV lamp (in the UVB range), results also in the production of superoxide radicals inside the bacterial cell, most likely due to photoactivation of aromatic amino acids and mainly tryptophan. Such radicals can target cellular components other than DNA, e.g., cell membrane, small molecules, and proteins that are essential for almost all cell activities, including repair of DNA dimers. The combination of DNA targeting photons (such as from LP UV lamp) with superoxide generating photons (higher wavelength, such as from MP UV or UVA/B LED lamps) thus results in much more effective inactivation of pathogens and lower recovery thereof. Such a combined system has both the benefits of LP(OH) systems, i.e. low power consumption and low heat production, and of MP systems, i.e. better inactivation, and reduced recovery.

### REFERENCES

Beck, S.E.; Rodriguez, R.A.; Linden, K.G.; Hargy, T.M.; Larason, T.C; Wright, H.B. Wavelength dependent UV inactivation and DNA damage of adenovirus as measured by cell culture infectivity and long range quantitative PCR. Environmental science & technology, 2014, 48(1), 591-598
Beck, S.E.; Ryu, H.; Boczek, L.A.; Cashdollar, J.L.; Jeanis, K.M.; Rosenblum, J.S.; Lawal, O.R.; Linden, K.G. Evaluating UV-C LED disinfection performance and investigating potential dual-wavelength synergy. Water Res. 2017, 109, 207-216.
Beck, S.E.; Wright, H.B.; Hargy, T.M.; Larason, T.C.; Linden, K.G. Action spectra for validation of pathogen disinfection in medium-pressure ultraviolet (UV) systems. Water Research, 2015, 70, 27-37
Chen, R.Z.; Craik, S.A.; Bolton, J.R. Comparison of the action spectra and relative DNA absorbance spectra of microorganisms: Information important for the determination of germicidal fluence (UV dose) in an ultraviolet disinfection of water. Water Research, 2009, 43(20), 5087-5096
Chen, J.; Loeb, S.; Kim, J.H. Critical review: LED revolution: fundamentals and prospects for UV disinfection applications. Environ. Sci.: Water Res. Technol. 2017, DOI: 10.1039/c6ew00241b.
Detsch, R.M.; Bryant, F.D.; Coohill, T.P. The wavelength dependence of herpes simplex virus inactivation by ultraviolet radiation. Photochemistry and photobiology, 1980, 32(2), 173-176
Gates F.L. A study of the bactericidal action of ultraviolet light. J Contam Hydrol. 1928, 13, 231-260
Gates, F.L. A study of the bactericidal action of ultra violet light III. The absorption of ultra violet light by bacteria. The Journal of general physiology, 1930, 14(1), 31-42
Harm, W. Biological effects of ultraviolet radiation. Press syndicate of the University of Cambridge, Cambridge, 1980
Jagger, J. Introduction to research in ultraviolet photobiology. Prentice-Hall, Eaglewood Cliffs, N.J., 1967
Lakretz, A.; Ron, E.Z.; Mamane, H. Biofouling control in water by various UVC wavelengths and doses, Biofouling, 2010, 26, 257-267
Lee, H.G.; Gu, M.B. Construction of a sodA:luxCDABE fusion Escherichia coli: comparison with a katG fusion strain through their responses to oxidative stresses, Appl. Microbiol. Biotechnol., 2003. 60, 577-580
Linden, K.; Shin, G.A.; Sobsey, M.D. Relative efficacy of UV wavelengths for the inactivation of Cryptosporidium parvum. Water Science and Technology, 2001, 43(12), 171-174
Mamane-Gravetz, H.; Linden, K.G.; Cabaj, A.; Sommer, R. Spectral sensitivity of Bacillus subtilis spores and MS2 coliphage for validation testing of ultraviolet reactors for water disinfection. Environmental science & technology, 2005, 39(20), 7845-7852
Meulemans, C.C.E. The basic principles of UV-disinfection of water, 1987
Moe, C. UV-C light emitting diodes, Radtech Report, 2014, 1, 45-49
Oguma, K.; Katayama, H.; Ohgaki, S. Photoreactivation of Escherichia coli after low- or medium-pressure UV disinfection determined by an endonuclease sensitive site assay. Appl. Environ. Microbiol. 2002, 68(12), 6029-6035
Sinha, R.P.; Hader, D.P. UV-induced DNA damage and repair: a review. Photochem. Photobiol. Sci. 2002, 1, 225-236
USEPA, 2006. Ultraviolet disinfection guidance manual for the final long term 2 enhanced surface water treatment rule, Environmental Protection. EPA 815-R-06-007
Wang, T.; MacGregor, S.J.; Anderson, J.G.; Woolsey, G.A. Pulsed ultra-violet inactivation spectrum of Escherichia coli. Water research, 2005, 39(13), 2921-2925
Water Environment Federation (WEF), 2015. Ultraviolet disinfection process concepts and equipment systems, in ultraviolet disinfection for wastewater. p. 17-29
Zimmer, J.L.; Slawson, R.M. Potential repair of Escherichia coli DNA following exposure to UV radiation from both medium- and low-pressure UV sources used in drinking water treatment. Appl. Environ. Microbiol. 2002, 68(7), 3293-3299

## Claims

1. A method for water disinfection comprising exposing water to a combination of at least two ultraviolet (UV) irradiation sources, for a sufficient period of time, wherein at least one of said UV irradiation sources comprises a low-pressure (LP) UV irradiation source emitting light at a wavelength of about 254nm, or at least one UV light-emitting diode (LED) irradiation source each independently emitting light at a wavelength of between 260nm and 275nm; at least another one of said UV irradiation sources comprises a sole UV LED irradiation source or a plurality of UV LED irradiation sources emitting light at either the same or different wavelengths of between 295nm and 305nm; and the exposure of said water to said at least two UV irradiation sources is carried out simultaneously.

2. The method of claim 1, wherein at least one of said UV irradiation sources comprises a LP UV irradiation source emitting light at a wavelength of about 254nm, or at least one UV LED irradiation source each independently emitting light at a wavelength of between 260nm and 270nm.

3. The method of claim 1, wherein:
(i) at least one of said UV irradiation sources comprises a LP UV irradiation source emitting light at a wavelength of about 254nm, or at least one UV LED irradiation source each independently emitting light at a wavelength of between 260nm and 270nm; and
(ii) at least another one of said UV irradiation sources comprises a sole UV LED irradiation source or a plurality of UV LED irradiation sources emitting light at either the same or different wavelengths of between 295nm and 300nm.

4. The method of claim 3, wherein:
(i) at least one of said UV irradiation sources comprises a LP UV irradiation source emitting light at a wavelength of about 254nm; and
(ii) at least another one of said UV irradiation sources comprises a sole UV LED irradiation source or a plurality of UV LED irradiation sources emitting light at either the same or different wavelengths of between 295nm and 300nm.

5. The method of any one of claims 1 to 4, wherein said sufficient period of time is up to 40 seconds.

6. The method of claim 5, wherein said sufficient period of time is up to 30 seconds.

7. The method of any one of claims 1-6, wherein said water is drinking water or wastewater effluents.

## Patentansprüche

1. Verfahren zur Desinfektion von Wasser, umfassend Exponieren von Wasser gegenüber einer Kombination von mindestens zwei Ultraviolett (UV)-Bestrahlungsquellen für einen ausreichenden Zeitraum, wobei mindestens eine der UV-Bestrahlungsquellen eine Niederdruck (ND)-UV-Bestrahlungsquelle, die Licht mit einer Wellenlänge von etwa 254 nm emittiert, oder mindestens eine Bestrahlungsquelle mit UV-Licht emittierender Diode (LED) umfasst, die jeweils unabhängig Licht mit einer Wellenlänge zwischen 260 nm und 275 nm emittiert; mindestens eine andere der UV-Bestrahlungsquellen eine einzelne UV-LED-Bestrahlungsquelle oder eine Vielzahl von UV-LED-Bestrahlungsquellen umfasst, die Licht mit entweder der gleichen oder unterschiedlichen Wellenlängen zwischen 295 nm und 305 nm emittieren;
und die Exposition des Wassers gegenüber den mindestens zwei UV-Bestrahlungsquellen simultan durchgeführt wird.

2. Verfahren nach Anspruch 1, wobei mindestens eine der UV-Bestrahlungsquellen eine ND-UV-Bestrahlungsquelle, die Licht mit einer Wellenlänge von etwa 254 nm emittiert, oder mindestens eine UV-LED-Bestrahlungsquelle umfasst, die jeweils Licht mit einer Wellenlänge zwischen 260 nm und 270 nm emittiert.

3. Verfahren nach Anspruch 1, wobei:
(i) mindestens eine der UV-Bestrahlungsquellen eine ND-UV-Bestrahlungsquelle, die Licht mit einer Wellenlänge von etwa 254 nm emittiert, oder mindestens eine UV-LED-Bestrahlungsquelle umfasst, die jeweils Licht mit einer Wellenlänge zwischen 260 nm und 270 nm emittiert; und
(ii) mindestens eine andere der UV-Bestrahlungsquellen eine einzelne UV-LED-Bestrahlungsquelle oder eine Vielzahl von UV-LED-Bestrahlungsquellen umfasst, die Licht mit entweder der gleichen oder unterschiedlichen Wellenlängen zwischen 295 nm und 300 nm emittieren.

4. Verfahren nach Anspruch 3, wobei:
(i) mindestens eine von den UV-Bestrahlungsquellen eine ND-UV-Bestrahlungsquelle umfasst, die Licht mit einer Wellenlänge von etwa 254 nm emittiert; und
(ii) mindestens eine andere der UV-Bestrahlungsquellen eine einzelne UV-LED-Bestrahlungsquelle oder eine Vielzahl von UV-LED-Bestrahlungsquellen umfasst, die Licht mit entweder der gleichen oder unterschiedlichen Wellenlängen zwischen 295 nm und 300 nm emittieren.

5. Verfahren nach einem der Ansprüche 1 bis 4, wobei der ausreichende Zeitraum bis zu 40 Sekunden beträgt.

6. Verfahren nach Anspruch 5, wobei der ausreichende Zeitraum bis 30 Sekunden beträgt.

7. Verfahren nach einem der Ansprüche 1 bis 6, wobei das Wasser Trinkwasser oder Abwasserströme ist.

## Revendications

1. Procédé de désinfection d'eau comprenant l'exposition d'eau à une combinaison d'au moins deux sources d'irradiation aux ultraviolets (UV), pendant un laps de temps suffisant, dans lequel au moins une desdites sources d'irradiation UV comprend une source d'irradiation UV à basse pression (BP) émettant de la lumière à une longueur d'onde d'environ 254 nm, ou au moins une source d'irradiation à diode électroluminescente (DEL) UV, chacune émettant indépendamment de la lumière à une longueur d'onde comprise entre 260 nm et 275 nm ; au moins une autre desdites sources d'irradiation UV comprend une seule source d'irradiation à DEL UV ou une pluralité de sources d'irradiation à DEL UV émettant de la lumière à des longueurs d'onde soit identiques ou différentes comprises entre 295 nm et 305 nm ; et l'exposition de ladite eau auxdites au moins deux sources d'irradiation UV est réalisée simultanément.

2. Procédé de la revendication 1, dans lequel au moins une desdites sources d'irradiation UV comprend une source d'irradiation UV BP émettant de la lumière à une longueur d'onde d'environ 254 nm, ou au moins une source d'irradiation à DEL UV, chacune émettant indépendamment de la lumière à une longueur d'onde comprise entre 260 nm et 270 nm.

3. Procédé de la revendication 1, dans lequel :
(i) au moins une desdites sources d'irradiation UV comprend une source d'irradiation UV BP émettant de la lumière à une longueur d'onde d'environ 254 nm, ou au moins une source d'irradiation à DEL UV, chacune émettant indépendamment de la lumière à une longueur d'onde comprise entre 260 nm et 270 nm ; et
(ii) au moins une autre desdites sources d'irradiation UV comprend une seule source d'irradiation à DEL UV ou une pluralité de sources d'irradiation à DEL UV émettant de la lumière à des longueurs d'onde soit identiques ou différentes comprises entre 295 nm et 300 nm.

4. Procédé de la revendication 3, dans lequel :
(i) au moins une desdites sources d'irradiation UV comprend une source d'irradiation UV BP émettant de la lumière à une longueur d'onde d'environ 254 nm ; et
(ii) au moins une autre desdites sources d'irradiation UV comprend une seule source d'irradiation à DEL UV ou une pluralité de sources d'irradiation à DEL UV émettant de la lumière à des longueurs d'onde soit identiques ou différentes comprises entre 295 nm et 300 nm.

5. Procédé de l'une quelconque des revendications 1 à 4, dans lequel ledit laps de temps suffisant va jusqu'à 40 secondes.

6. Procédé de la revendication 5, dans lequel ledit laps de temps suffisant va jusqu'à 30 secondes.

7. Procédé de l'une quelconque des revendications 1 à 6, dans lequel ladite eau est de l'eau potable ou des effluents d'eaux usées.
